# EUROPÄISCHE PATENTANMELDUNG

(11) **EP 1 159 919 A2**
(43) Veröffentlichungstag der Anmeldung: **05.12.2001**
(21) Anmeldenummer: 01112817.0
(22) Anmeldetag: 30.05.2001
(51) Int. Cl.: A61B 17/04

(54) **Vorrichtung zum Verschliessen einer Öffnung in einer Gewebeschicht**

(30) Priorität: 31.05.2000 DE 10027186
(71) Anmelder: Aesculap AG & Co. KG, 78532 Tuttlingen (DE)
(72) Erfinder: Müller, Gottfried, 97999 Igersheim (DE); Dworschak, Manfred, 78589 Dürbheim (DE)
(74) Vertreter: Böhme, Ulrich, Dr. Dipl.-Phys.

(57) **Zusammenfassung**

Um bei einer Vorrichtung zum Verschließen eines Stichkanals in einer Gewebeschicht mit einem chirurgischen Nähfaden in einfacher Weise die Ausbildung einer Bruchlücke an der Innenseite der Gewebeschicht zu verhindern, wird vorgeschlagen, daß die Vorrichtung ein flächiges, zusammenfaltbares Anlageelement umfaßt, an dem der Nähfaden derart angreift, daß sich das Anlageelement bei Zug an dem durch den Stichkanal der Gewebeschicht laufenden Nähfaden entfaltet und flächig an den Rand des Stichkanals anlegt.

## Beschreibung

Die Erfindung betrifft eine Vorrichtung zum Verschließen einer Öffnung in einer Gewebeschicht mit einem chirurgischen Nähfaden.

Bei videolaparoskopischen Eingriffen sind Trokarzugänge mit einem Durchmesser von mindestens 10 mm üblich. Nach Beendigung laparoskopischer Operationen werden die für die Trokarhülsen notwendigen Stichkanäle meist nur mit einer Hautnaht versorgt, die Faszie und das Peritoneum werden nicht berücksichtigt. Dadurch kann es zu Narbenhernien kommen, bei denen die Faszienläsion, die durch den Trokareinstich verursacht wurde, eine Bruchlücke bildet, in die sich das Peritoneum vorwölbt und in die sich Darmsegmente einklemmen können.

Es ist daher vorteilhaft, wenn beim Verschließen der Stichkanäle auch Faszie und Peritoneum mit einer Naht versorgt werden. Dies ist allerdings ein relativ komplizierter Vorgang, bei dem auch das Risiko von zusätzlichen Verletzungen besteht.

Es ist Aufgabe der Erfindung, eine Vorrichtung zum Verschließen eines Stichkanals in einer Gewebeschicht vorzuschlagen, welche ohne komplizierte Naht in Faszie und Peritoneum den Durchstichkanal innenseitig so verschließt, daß die Gefahr einer Bruchlücke beseitigt wird.

Diese Aufgabe wird bei einer Vorrichtung der eingangs beschriebenen Art erfindungsgemäß dadurch gelöst, daß sie ein flächiges, zusammenfaltbares Anlageelement umfaßt, an dem der Nähfaden derart angreift, daß sich das Anlageelement bei Zug an den beiden durch den Stichkanal laufenden Enden des Nähfadens entfaltet und flächig an den Rand des Stichkanals anlegt.

Dadurch ist es möglich, das Anlageelement in gefaltetem Zustand von außen nach innen durch den Stichkanal hindurchzuführen, zum Beispiel durch die in den Stichkanal eingesetzte Trokarhülse hindurch, bis das Anlageelement an der Innenseite der Gewebeschicht angeordnet ist. Durch Zug an dem am Anlageelement angreifenden Nähfaden wird das Anlageelement entfaltet und legt sich innenseitig an den Stichkanal an, der auf diese Weise verschlossen wird. Die durch den Stichkanal nach außen geführten Enden des Nähfadens können dann verwendet werden, um die Hautnaht auszuführen, dabei spannt der Nähfaden das aufgefaltete flächige Anlageelement gegen die Innenseite der Gewebeschicht und preßt dort Faszie und Peritoneum den Durchstichkanal verschließend gegeneinander.

Der Nähfaden kann in verschiedener Weise an dem Anlageelement angreifen, beispielsweise kann der Nähfaden einstückig mit dem Anlageelement ausgebildet oder mit diesem durch Verklebung oder Verschweißung verbunden sein, bei einer besonders bevorzugten Ausführungsform ist vorgesehen, daß das Anlageelement mindestens eine Öffnung aufweist, durch die der Nähfaden hindurchgeführt ist. Beispielsweise könnte der Nähfaden durch eine zentrale Öffnung hindurchgeführt und auf der Rückseite verknotet sein.

Bei einer besonders bevorzugten Ausführungsform ist vorgesehen, daß das Anlageelement eine Faltlinie aufweist sowie drei Öffnungen für den Nähfaden, von denen zwei auf gegenüberliegenden Seiten der Faltlinie liegen und eine im Bereich der Faltlinie, daß der Nähfaden auf einer äußeren Seite des Anlageelements zwischen den beiden äußeren Öffnungen verläuft, diese durchsetzt und auf der gegenüberliegenden inneren Seite bis zu der mittleren Öffnung zurückläuft, und daß beide Enden des Fadens durch die mittlere Öffnung auf die äußere Seite des Anlageelements zurückgeführt sind. Damit umgibt der Nähfaden das Anlageelement nach Art einer Schleife und ist im Zentrum wieder zurückgeführt, so daß bei einem Zug im Zentrum eine Auffaltung des Anlageelements erfolgt.

Das Anlageelement kann insbesondere aus einem resorbierbaren Kunststoffmaterial bestehen.

Es ist günstig, wenn das Anlageelement eine Dicke zwischen 0,1 mm und 1 mm aufweist, vorzugsweise in der Größenordnung von 0,5 mm. Das Anlageelement kann beispielsweise eine dünne Scheibe mit einer Dicke von 0,1 mm sein, die im Bereich einer zentralen Öffnung und im äußeren Randbereich dicker ausgebildet ist, beispielsweise in der Größenordnung von 0,3 mm und 0,5 mm, so daß dadurch eine Formstabilisierung erfolgt.

Weiterhin ist vorteilhaft, wenn das Anlageelement in seiner aufgefalteten Lage mindestens in einer Richtung eine Querabmessung von 12 bis 50 mm aufweist, vorzugsweise in der Größenordnung von 20 mm.

Dabei kann das Anlageelement unterschiedliche Querschnittsformen haben, es kann beispielsweise kreisförmig, oval, rechteckig oder rautenförmig sein, wesentlich ist lediglich, daß das Anlageelement den Durchstichkanal überdeckt.

Es ist weiterhin vorgesehen, daß gemäß einer bevorzugten Ausführungsform der Nähfaden an beiden Enden mit Nadeln verbunden ist. Dadurch ist es möglich, diesen Nähfaden unmittelbar zur Ausbildung der Hautnaht zu verwenden.

Es ist dabei günstig, wenn die Nadeln gebogen sind.

Bei einer besonders bevorzugten Ausführungsform umfaßt die Vorrichtung zur Aufnahme des Anlageelements ein rohrförmiges Einführinstrument, in welchem das Anlageelement in gefaltetem Zustand angeordnet ist und durch welches die Endes des Nähfadens nach außen geführt sind. Dieses Einführinstrument kann durch den Stichkanal in das Körperinnere eingeführt werden, und durch dieses Einführinstrument läßt sich das gefaltete Anlageelement durch den Stichkanal hindurchführen.

Weiterhin ist günstig, wenn in dem Einführinstrument ein Ausschiebeelement in Längsrichtung verschiebbar ist, mit dessen Hilfe das Anlageelement aus dem Inneren des Einführinstruments in das Körperinnere ausstoßbar ist.

Das Ausschiebeelement kann einen Durchtrittsquerschnitt für den Nähfaden ausbilden, beispielsweise einen seitlichen Rücksprung oder insbesondere Längskanäle im Ausschiebeelement selbst.

Das Ausschiebeelement kann ein in das Einführinstrument eintauchender Stopfen sein.

Es ist günstig, wenn der Außendurchmesser des Einführinstruments so gewählt ist, daß es in übliche Trokarhülsen für die laparoskopische Operationstechnik einführbar ist. Auf diese Weise kann am Ende der Operation in die noch eingesetzte Trokarhülse das Einführinstrument eingeschoben werden, aus welchem dann das gefaltete Anlageelement in den Körperinnenraum ausgeschoben wird. Nach Entfernung des Einführinstruments und der Trokarhülse läßt sich dann das Anlageelement nach Auffalten innenseitig an die Gewebeschicht anlegen, so daß der Stichkanal verschlossen wird.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht eines in eine Trokarhülse eingesetzten Einführinstruments mit einem in diesem angeordneten, gefalteten Anlageelement;
- Figur 2:: eine vergrößerte Teilansicht des distalen Endes des Einführinstrumentes der Figur 1 mit ausgeschobenem Anlageelement;

- Figur 3:: eine Ansicht ähnlich Figur 2 nach Anlage des Anlageelementes an der Gewebeschicht und beim Auffalten des Anlageelementes;
- Figur 4:: eine Schnittansicht des zu verschließenden Stichkanals nach Entfernung von Trokarhülse und Einführinstrument und nach Anlage des Anlageelementes an der Innenseite der Gewebeschicht und
- Figur 5:: eine Ansicht ähnlich Figur 4 nach Ausbildung einer Hautnaht.

In der Zeichnung ist eine Gewebeschicht 1 mit einer Oberhautschicht 2, einer Fettschicht 2a, einer Muskelschicht 3, der Faszie 4 und dem Peritoneum 5 schematisch dargestellt, durch diese Gewebeschicht 1 führt ein diese quer durchsetzender Stichkanal 6, durch den eine Trokarhülse 7 hindurchgeschoben ist. Diese Trokarhülse 7 ragt üblicherweise durch die gesamte Gewebeschicht 1 hindurch und bildet einen Zugang zum Körperinneren, durch den im Inneren Operationsschritte durchgeführt werden können. Die Gewebeschicht 1 kann beispielsweise die Bauchdecke sein, so daß ein Zugang zum Bauchraum geschaffen wird.

In die Trokarhülse 7 ist ein längliches Einführinstrument 8 eingeschoben, welches ein längliches Rohr 9 umfaßt, in dem ein stopfenförmiges Ausschiebeelement 10 mit Hilfe einer Schubstange 11 in Längsrichtung des Rohres 9 verschiebbar ist. Die Schubstange 11 ragt aus dem Rohr 9 am proximalen Ende heraus und trägt dort einen Griff 12.

Auf der distalen Seite des Ausschiebeelementes 10 befindet sich im Inneren des Rohres 9 ein zusammengefaltetes plättchenförmiges Anlageelement 13. Es handelt sich dabei im dargestellten Ausführungsbeispiel um ein kreisförmiges Plättchen mit einem Durchmesser von beispielsweise 20 mm und einer Dicke von beispielsweise 0,5 mm, dieses Plättchen besteht aus einem resorbierbaren Kunststoffmaterial und ist so zusammengefaltet, daß es in dem Rohr 9 des Einführinstrumentes 8 aufgenommen ist. Dieses Rohr 9 hat einen Außendurchmesser von beispielsweise 8 mm, während die in der Zeichnung dargestellte Trokarhülse 7 beispielsweise einen Außendurchmesser von 10 mm haben kann.

Das Anlageelement 13 ist in der Mitte längs einer Faltlinie 14 zusammengefaltet und weist eine zentrale Öffnung 15 und auf gegenüberliegenden Seiten der Faltlinie 14 im Abstand zu dieser zusätzlich jeweils eine weitere Öffnung 16 beziehungsweise 17 auf. Ein chirurgischer Nähfaden 18 ist mit einem Ende 19 zunächst durch die zentrale Öffnung 15 hindurchgesteckt und verläuft längs der innenliegenden Seitenfläche 20 zu einer der äußeren Öffnungen 16, durchsetzt diese und verläuft dann an der außenliegenden Seitenfläche 21 des Anlageelementes 13 zu der anderen außenliegenden Öffnung 17. Durch diese tritt der Nähfaden 18 hindurch und verläuft an der innenliegenden Seitenfläche 20 entlang zurück zur zentralen Öffnung 15, durch die der Nähfaden 18 mit dem nunmehr anderen Ende 22 hindurchgesteckt ist. Die beiden Enden 19 und 22 des Nähfadens 18 treten durch Längskanäle 23 in dem stopfenförmigen Ausschiebeelement 10 hindurch und verlaufen dann bis zum proximalen Ende des Rohres 9, wo sie mit außerhalb des Rohres 9 angeordneten, gebogenen Nadeln 24, 25 verbunden sind.

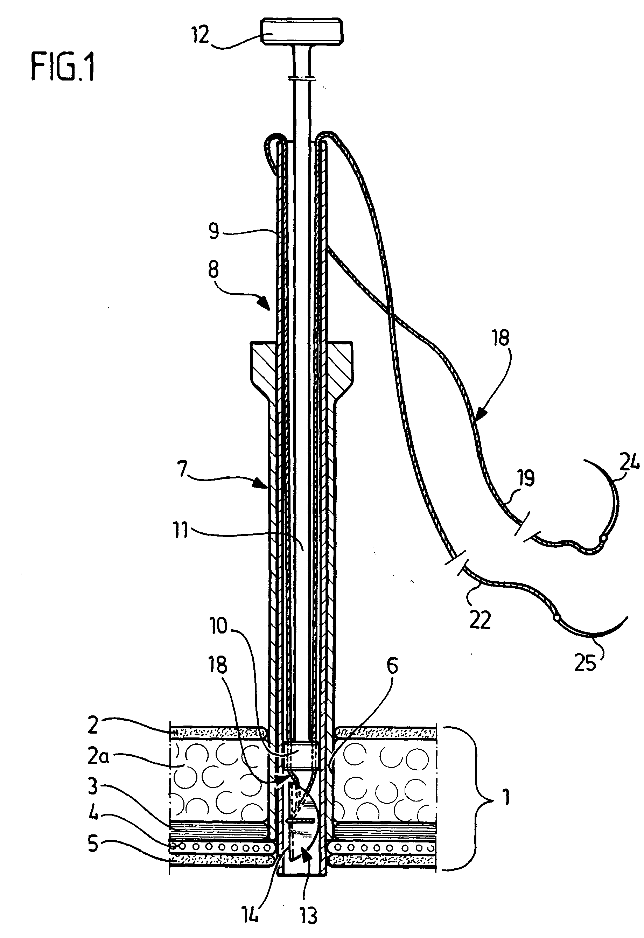

Um den Stichkanal 6 zu verschließen, wird zunächst in die in den Stichkanal 6 eingesetzte Trokarhülse 7 das Einführinstrument 8 eingeschoben, bis dessen distales Ende die Gewebeschicht 1 durchsetzt. Anschließend wird die Trokarhülse 7, die normalerweise relativ weit in das Körperinnere hineinragt, so weit nach außen verschoben, daß deren distales Ende innerhalb der Gewebeschicht 1 endet, wie dies in den Figuren 1 bis 3 dargestellt ist.

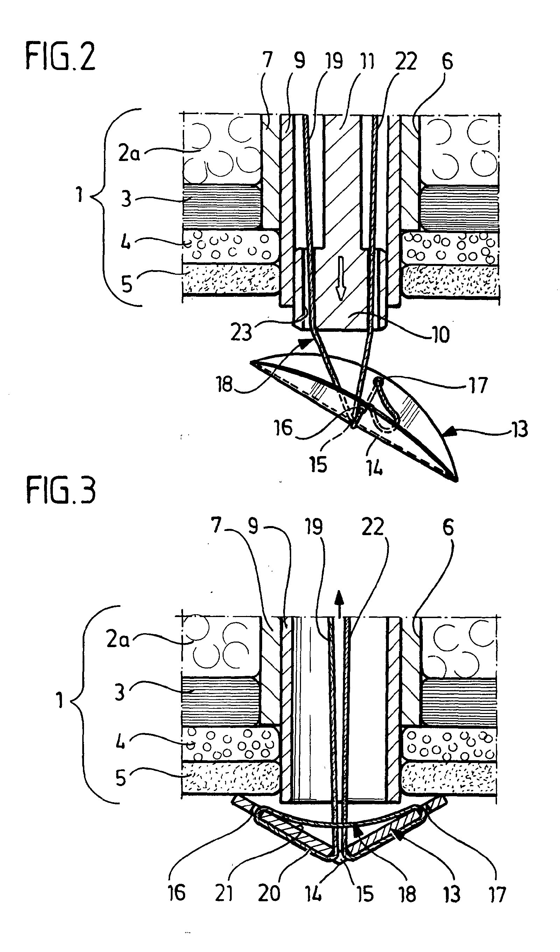

Durch Verschieben der Schubstange 11 und des daran gehaltenen Ausschiebeelementes 10 wird das im Inneren des Rohres angeordnete, zusammengefaltete Anlageelement 13 aus diesem ausgeschoben und gelangt in das Körperinnere, wo sich das Anlageelement 13 auffalten kann, wie dies in Figur 2 dargestellt ist.

Durch Zug an den Enden 19 und 22 des Nähfadens 18 läßt sich das Anlageelement 13 an die Innenseite der Gewebeschicht 1 anlegen, und durch weiteren Zug wird das Anlageelement 13 vollständig aufgefaltet, da der Nähfaden zentral an dem Anlageelement angreift, wie dies aus der Darstellung der Figur 3 deutlich wird. Das Anlageelement 13 legt sich somit flächig an die Innenseite der Gewebeschicht 1 an und überdeckt den Stichkanal 6.

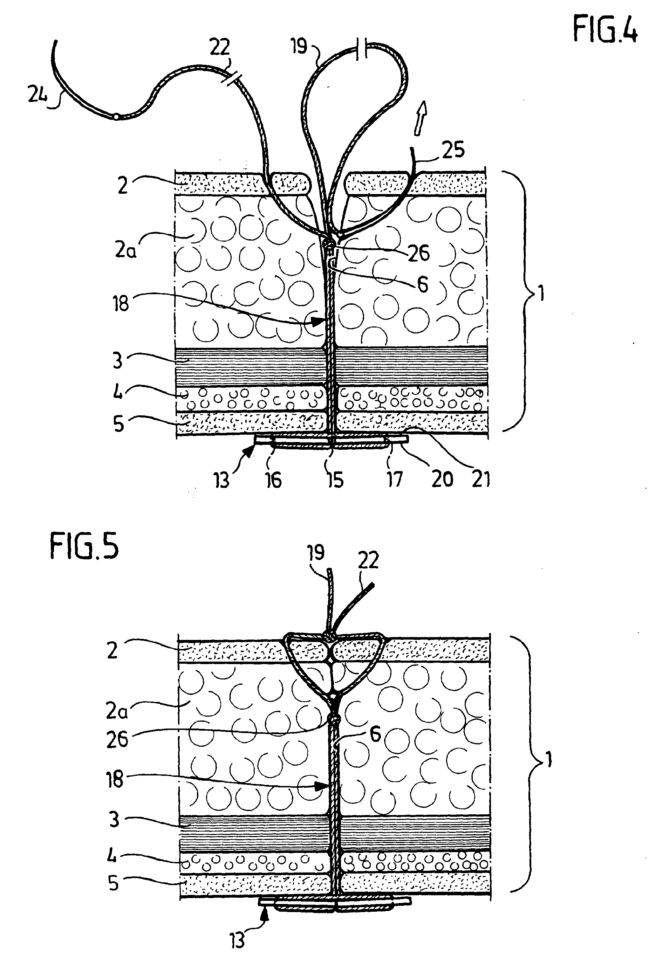

Nacheinander werden nun die Trokarhülse und das Einführinstrument 8 aus dem Stichkanal 6 herausgezogen, so daß im Stichkanal lediglich der Nähfaden 18 verbleibt. Der Stichkanal 6 schließt sich dabei, und durch Zug am Nähfaden 18 läßt sich nunmehr der Stichkanal innenseitig durch das Anlageelement vollständig überdecken, das durch Zug an die Innenseite der Gewebeschicht 1 angelegt wird. Es ist günstig, in dieser Lage die beiden Enden 19 und 22 zu verknoten, wobei der dabei gebildete Knoten 26 in der unter der Oberhautschicht 2 angeordneten Fettschicht 2a angeordnet wird (Figur 4).

Mit Hilfe der Nadeln 24 und 25 werden dann die Enden 19 und 22 durch einen vom Stichkanal nach außen geführten Einstich in die Fettschicht 2a und durch die Oberhautschicht 2 hindurch und außenseitig verknotet, so daß der obere Teil der Fettschicht 2a und der Oberhautschicht 2 gegeneinandergezogen werden, der Stichkanal 6 wird dadurch außenseitig verschlossen (Figur 5).

Es ist damit sichergestellt, daß mit Hilfe des Nähfadens, der zur Herstellung der Hautnaht in jedem Fall notwendig ist, zusätzlich auch eine Überdeckung und ein Verschluß des Stichkanals 6 auf der Innenseite erfolgt, ohne daß dazu zusätzliche Arbeitsschritte notwendig sind, dies erfolgt allein durch das Anspannen des Nähfadens und die dadurch erzielte flächige Anlage des Anlageelementes an der Gewebeschicht 1.

## Patentansprüche

1. Vorrichtung zum Verschließen eines Stichkanals (6) in einer Gewebeschicht (1) mit einem chirurgischen Nähfaden (18), **dadurch gekennzeichnet, daß** sie ein flächiges, zusammenfaltbares Anlageelement (13) umfaßt, an dem der Nähfaden (18) derart angreift, daß sich das Anlageelement (13) bei Zug an dem durch den Stichkanal (6) der Gewebeschicht (1) laufenden Nähfaden (18) entfaltet und flächig an den Rand des Stichkanals (6) anlegt.

2. Vorrichtung nach Anspruch 1, **dadurch gekennzeichnet, daß** das Anlageelement (13) mindestens eine Öffnung (15, 16, 17) aufweist, durch die der Nähfaden (18) hindurchgezogen ist.

3. Vorrichtung nach Anspruch 2, **dadurch gekennzeichnet, daß** das Anlageelement (13) eine Faltlinie (14) aufweist sowie drei Öffnungen (15, 16, 17) für den Nähfaden (18), von denen zwei Öffnungen (16, 17) auf gegenüberliegenden Seiten der Faltlinie (14) liegen und eine Öffnung (15) im Bereich der Faltlinie (14), daß der Nähfaden (18) auf einer außenliegenden Seitenfläche (21) des Anlageelemente (13) zwischen den beiden äußeren Öffnungen in (16, 17) verläuft, diese durchsetzt und auf der gegenüberliegenden innenliegenden Seitenfläche (20) bis zu der mittleren Öffnung (15) zurückläuft, und daß beide Enden (19, 22) des Nähfadens (18) durch die mittlere Öffnung (15) auf die außenliegende Seitenfläche (21) des Anlageelementes (13) zurückgeführt sind.

4. Vorrichtung nach einem der voranstehenden Ansprüche, **dadurch gekennzeichnet, daß** das Anlageelement (13) aus resorbierbarem Kunststoffmaterial besteht.

5. Vorrichtung nach einem der voranstehenden Ansprüche, **dadurch gekennzeichnet, daß** das Anlageelement (13) eine Dicke zwischen 0,1 mm und 1 mm aufweist.

6. Vorrichtung nach einem der voranstehenden Ansprüche, **dadurch gekennzeichnet, daß** das Anlageelement (13) in seiner aufgefalteten Lage mindestens in einer Richtung eine Querabmessung von 12 bis 50 mm aufweist.

7. Vorrichtung nach einem der voranstehenden Ansprüche, **dadurch gekennzeichnet, daß** der Nähfaden (18) an beiden Enden (19, 22) mit Nadeln (24 beziehungsweise 25) verbunden ist.

8. Vorrichtung nach Anspruch 7, **dadurch gekennzeichnet, daß** die Nadeln (24, 25) gebogen sind.

9. Vorrichtung nach einem der voranstehenden Ansprüche, **dadurch gekennzeichnet, daß** sie zur Aufnahme des Anlageelementes (13) ein rohrförmiges Einführinstrument (8) umfaßt, in welchem das Anlageelement (13) im gefalteten Zustand angeordnet ist und durch welches die Enden (19, 22) des Nähfadens (18) nach außen geführt sind.

10. Vorrichtung nach Anspruch 9, **dadurch gekennzeichnet, daß** in dem Einführinstrument (8) ein Ausschiebeelement (10) in Längsrichtung verschiebbar ist.

11. Vorrichtung nach Anspruch 10, **dadurch gekennzeichnet, daß** das Ausschiebeelement (10) einen Durchtrittsquerschnitt (23) für den Nähfaden (18) ausbildet.

12. Vorrichtung nach Anspruch 11, **dadurch gekennzeichnet, daß** der Durchtrittsquerschnitt (23) durch Längskanäle im Ausschiebeelement (10) gebildet wird.

13. Vorrichtung nach einem der Ansprüche 10 bis 12, **dadurch gekennzeichnet, daß** das Ausschiebeelement (10) ein in das Einführinstrument (8) eintauchender Stopfen ist.

14. Vorrichtung nach einem der Ansprüche 10 bis 13, **dadurch gekennzeichnet, daß** der Außendurchmesser des Einführinstrumentes (8) so gewählt ist, daß es in üblichen Trokarhülsen (7) für die laparoskopische Operationstechnik einführbar ist.
